# EUROPEAN PATENT APPLICATION

(11) **EP 4 759 918 A1**
(43) Date of publication of application: **17.06.2026**
(21) Application number: 24850406.0
(22) Date of filing: 07.08.2024
(51) Int. Cl.: C12N 1/10, G01N 33/48, C12Q 1/02

(54) **IN VITRO METHOD FOR QUANTIFYING AND ASSESSING THE VIABILITY OF OOCYSTS OF EIMERIA SPP**

(30) Priority: 09.08.2023 BR 102023015995
(71) Applicant: Laboratório Bio-Vet Ltda., 06730-000 Vargem Grande Paulista (SP) (BR)
(72) Inventor: MARIA PEREIRA, Ana, São Paulo (BR); TAKAO YOSHIDA, Jony, São Paulo (BR); FERNANDEZ, Sandra, São Paulo (BR)
(74) Representative: Bryn Aarflot AS
(86) International application number: PCT/BR2024/050346
(87) International publication number: WO 2025/030228

(57) **Abstract**

This document belongs to the field of Biology and Veterinary Medicine, presenting a new *in vitro* and simplified method for differentiation, quantification and viability assessment of *Eimeria oocysts* spp using an iodine-based reagent.

## Description

### FIELD OF THE INVENTION

This document belongs to the field of Biology and Veterinary Medicine, presenting a new and simplified *in vitro method for* differentiating, quantifying and evaluating the viability of *Eimeria* spp. oocysts using an iodine-based reagent.

### STATE OF TECHNICAL DESCRIPTION

Eimeriosis or coccidiosis is a common disease in animals caused by protozoa of the genus *Eimeria,* being more common particularly in poultry. It is an infection that usually affects young animals and can lead to the death of entire aviaries, directly impacting the economy of rural producers.

Thus, the current main strategy for preventing coccidiosis in these animals is through flock vaccination. Vaccines against coccidiosis are produced from the oocysts of the parasite *Eimeria* spp.

Currently, the viability analysis of *Eimeria oocysts* is done by inoculating the oocyst suspension in birds and waiting for the incubation time of the oocysts in the birds in order to verify the pathological effects caused by the inoculated oocysts.

When the oocysts are not viable, the birds do not show any degree of intensity of the pathological effects of the oocysts compared to non-inoculated birds. Such a technique is expensive due to the need for an area to handle these animals to obtain a result after days, in addition to presenting a challenge because it is a method of testing in animals in *vivo.*

Furthermore, the viability evaluation method currently used in the market presents qualitative results, and it is not possible to quantify the viability of the oocysts in percentage for their subsequent application in obtaining APIs for the production of vaccines to prevent coccidiosis, a very common disease in birds and which can lead to significant losses in industrial poultry farming.

### BACKGROUND OF THE INVENTION

US Patent 6,344,340 B1 discloses a viability assay for sporocyst-forming protozoa, in which a sample of the protozoan is treated with at least one vital dye, in order to determine the viability of the protozoan from the differentiation of the color by means of microscopy of fulorescence. Therefore, it completely diverges from the method proposed by the search object, which proposes a colorimetric method using an iodine-based reagent to identify viable *Eimeria* oocysts in a more simplified way, by means of a counting chamber in an optical microscope.

Patent document IL176475A discloses a method for purifying *Eimeira* spp. from fecal material using enzymes. The method consists of putting the fecal material in contact with polysaccharidase, such as cellulase and pectinase, and later amylase, in order to remove such material. Therefore, it completely diverges from the method proposed by the claimed invention, which proposes a colorimetric method using an iodine-based reagent to identify viable *Eimeria* oocysts, as well as their differentiation from other particles, such as starch.

The patent document AU200031298A discloses a method of identification of oocysts involving antibody binding, in addition to using another species of protozoan (*Cryptosporidium* spp.). Therefore, it completely diverges from the method proposed by the present invention, which proposes a colorimetric method using an iodine-based reagent for the identification of viable oocysts of *Eimeria.*

Patent US7229615B2 discloses a method of producing vaccines, in which part of the claims refers to sporulation and separation of Eimeria oocysts for the production of such vaccines. More specifically, the described method uses water and hydrogen peroxide, and subsequent aeration of the material and pH conditioning to recover the material's oocysts. Therefore, it completely diverges from the method proposed by the present invention, which proposes a colorimetric method using an iodine-based reagent for the identification of viable oocysts of *Eimeria.*

Patent document CN113367099A reveals the use of iodine solution in eggs in a method for obtaining coccidiosis-free birds to be used in an experimental field. Therefore, it completely diverges from the method proposed by the search object, which proposes a colorimetric method using an iodine-based reagent to identify viable oocysts of *Eimeria.*

The article "A simple and efficient method for isolation of a single Eimeria oocyst from poultry litter using a micromanipulator" (Dongjean Yim et al., 2011) reveals the use of a micromanipulator, as well as genomic identification for the isolation of a single Eimeria oocyst from chicken fecal material. Therefore, it completely diverges from the method proposed by the present invention, which proposes a colorimetric method using an iodine-based reagent to identify viable oocysts of Eimeria.

The article "Single oocyst infection: a simple method for isolation of Eimeria spp. from the mixed field samples" (RE Khalafalla et al., 2010) reveals two simple methods of morphological identification of sporulated oocysts of *Eimeria* spp. Isolated. The first describes the use of gelatin, and the second describes the DNA extraction method for identifying oocysts. Therefore, it completely diverges from the method proposed by the search object, which proposes a colorimetric method using an iodine-based reagent to identify viable oocysts of *Eimeria.*

Therefore, the use of an iodine-based solution for the differentiation, quantification and viability assessment of viable oocysts of *Eimeria* spp., in a simplified and totally *in vitro method, is not described in the state of the art.*

### SUMMARY OF THE INVENTION

The present invention is presented and characterized in independent claims, while the dependent claims describe other characteristics of the invention or modalities related to the main inventive idea.

In a first aspect, the present invention claims the use of an iodine-based solution in the *in vitro quantification and evaluation* of the viability of *Eimeria* spp. oocysts, using a counting chamber in an optical microscope.

In a second aspect, the invention claims that the iodine solution used has a formulation of 0.4% to 3% of resublimated iodine; 1.8% to 5% potassium iodide; 70% to 85% alcohol; 25% to 35% purified water.

In a third aspect, the invention claims that the differentiation of oocysts takes place from the differentiation of stained and unstained particles and the viable oocysts become translucent, while the starch particles and/or non-viable oocysts are stained. Additionally, the present invention provides that the counting can be performed manually and/or using an image analysis system.

In a fourth aspect, the invention claims that the optical microscope counting chambers used may be a Neubouer, Fuchs Rosenthal, Petroff-Hausser, Sedgwick-Rafter or Burker chamber.

In a fifth aspect, the present invention claims that the methodology can be applied in the verification of the status of oocyst bulks, in the definition of the stability time of Active Pharmaceutical Ingredients and of vaccines, in the quality control of the manufacturing process of vaccines against coccidiosis, in the evaluation of improvements in the production process, in the estimation of the level of vaccine response to the vaccine against coccidiosis *in vitro .*

### OBJECTIVES OF THE INVENTION

The main objective of the present invention is to propose an innovative and simplified technique for the quantitative evaluation of viable oocysts of *Eimeria* spp., in addition to their differentiation from other particles and non-oocysts (such as starch, for example) through the staining of oocysts in suspension using iodine-based reagent.

Another objective of the present invention is to optimize the process of evaluating the viability of oocysts, without the need to carry out tests on animals, a possible method to be carried out *in vitro.*

Another objective of the present invention is to facilitate the counting of oocysts with the presence of a high amount of starch in the suspensions and the reduction of analytical errors in the differentiation of oocysts from non-oocysts, in addition to facilitating the quantification of the suspensions.

### BRIEF DESCRIPTION OF THE FIGURES

The characteristics, aspects and advantages of the present invention will be better understood in relation to the following description and its claims from the attached figures, in which:

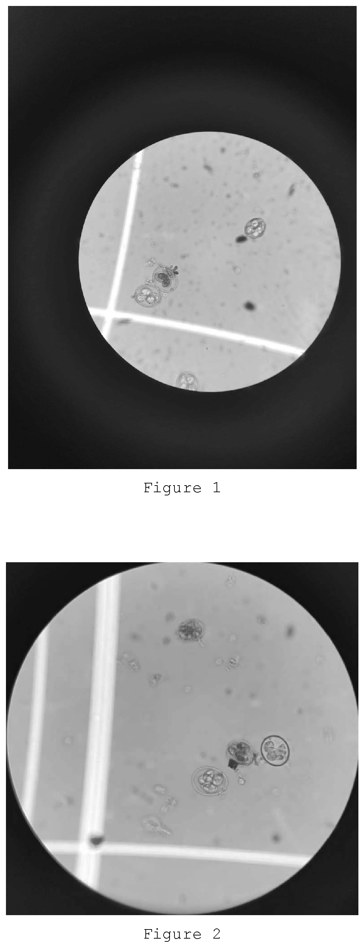

Figures 1 and 2 demonstrate the visualization of the difference between stained and unstained oocysts under an optical microscope.

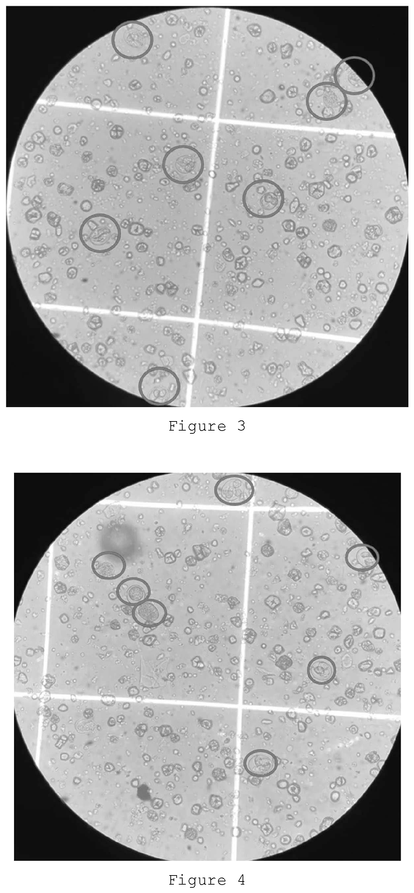

Figures 3 and 4 demonstrate conventional oocyst quantification.

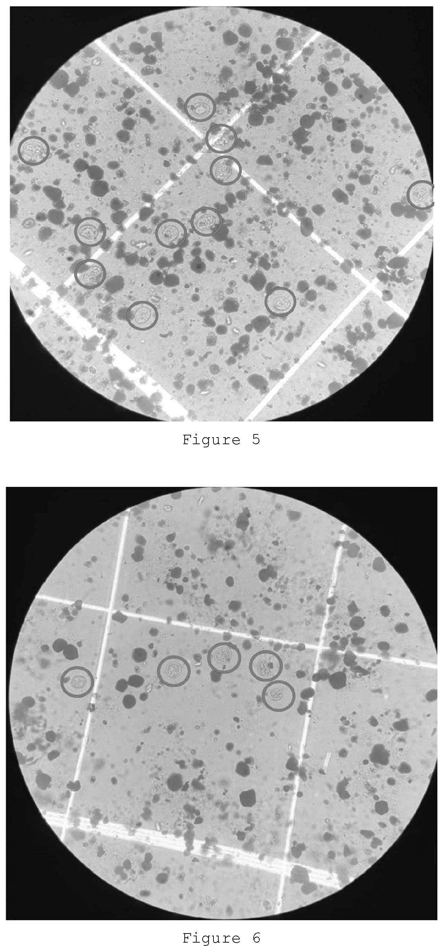

Figures 5 and 6 demonstrate the quantification of oocysts using the Iodine solution.

### DETAILED DESCRIPTION OF THE INVENTION

The present description aims to deepen the details on the inventive concept, provide examples that facilitate its understanding and provide precise technical data on some of the ways of implementing the inventive concept of the invention. The detailed description also aims to avoid the repetition, by third parties of the extensive experimentation, financial investments, time and intellectual activity that the inventors/depositors made to solve the technical problems now solved.

Currently, oocyst counting is performed using the Neubouer chamber for process control and for the formulation of vaccines against coccidiosis. The vaccine manufacturing process is based on collecting feces from birds infected with oocysts.

Due to the matrix where the oocysts are found (bird feces), several particles are found that considerably affect the identification of oocysts, mainly the presence of starch. To improve the identification of oocysts in relation to these particles, and also to differentiate viable from non-viable oocysts, a reagent containing iodine was added.

Iodine binds to starch molecules, causing their dark color, facilitating the differentiation of starch particles, which become dark, from oocysts, which become translucent.

With this technique, it is also possible to observe that some oocysts are stained less intensely than starch. It is also observed that such stained oocysts show characteristic deformations of non-viable oocysts.

After several experiments and analysis of samples, it was observed that other oocysts, despite their normal appearance, were being stained by the reagent. Furthermore, when performing a forced oocyst inactivation procedure, it was possible to assess the correlation between the percentage of viability loss and the stained oocysts. In this way, it was possible to verify that the claimed method is useful for differentiating starch oocysts, and also for evaluating the viability of oocysts.

The iodine solution used for the methods claimed by the present invention has the following formulation:
- 0.4% to 3% resublimated iodine;
- 1.8% to 5% potassium iodide;
- 70% to 85% alcohol;
- 25% to 35% purified water.

The first method using the aforementioned iodine solution claimed in the present invention is carried out by a simple procedure, whose steps are described below:
The. Dilute the Iodine solution in distilled water in a proportion between 1:0.2 and 1:20 at a temperature of 15-30°C at the time of use;
B. Aliquot the diluted iodine solution into tubes;
w. Keep the *Eimeria* suspension to be analyzed in the temperature range between 15°C and 30°C;
d. Homogenize the *Eimeria* suspension and transfer it to the tube containing the Iodine solution from a);
It is. Optionally dilute the *Eimeria* suspension in serial dilutions using the iodine solution when necessary;
f. Wait for up to 35 minutes;
g. Quantify the difference between stained and unstained oocysts in an optical microscope counting chamber, such as a Neubauer chamber.

The second method claimed in the present invention is carried out by the steps described below:
i) Dilute the Iodine Solution between 1:0.1 and 1:10 in distilled water refrigerated between 2 and 8°C at the time of use;
ii) Aliquot the diluted Iodine solution into bottles suitable for serial dilution;
*Eimeria* suspension to be analyzed refrigerated at a temperature range between 2 and 8°C;
iv) Homogenize the *Eimeria suspension* ;
v) Transfer the suspension to the flask containing the iodine solution (1:1) and then continue in serial dilution using the iodine solution until the ideal dilution of the study;
vi) Quantify the number of oocysts of *Eimeria* spp. found.

The method described above allows the differentiation of oocysts of *Eimeria* spp. from non-oocysts by facilitating the counting of oocysts with a high amount of starch in the suspensions, enabling improvements in the counting process and reduction of analytical errors in differentiation.

Both aforementioned methods provide quantitative information that can be useful in the following situations:
Check the status of oocyst bulks (IFAs) before their formulation, adjusting the formula according to the % of viability, allowing the production of vaccines that have the same quality levels in different batches produced.

Define stability time of APIs and vaccines according to temperature and storage conditions.

Check the storage of the APIs and vaccines produced (which are negatively affected by the storage temperature), if they were at the ideal temperature or if they were exposed to temperatures outside the specification.

Control of the manufacturing process of vaccines against coccidiosis, being used to assess the loss of viability during the production process, in order to help validate the decontamination of surfaces, materials and processes.

Evaluate improvements in the production process due to the inclusion of solutions to reduce the loss of viability.

Estimate the level of vaccine response without the need for animal testing.

## Claims

1. *In vitro* method for evaluating the viability of *Eimeria oocysts* spp. comprising the steps of
a) Sampling a volume of suspension of oocysts of *Eimeria* spp. and mix with a volume of iodine-based solution,
b) Adding the mixture obtained in a) to a counting chamber,
c) Counting the oocysts of *Eimeria* spp. stained and unstained using an optical microscope,
d) Defining oocyst viability.

2. *In vitro* method for quantification of *Eimeria oocysts* spp. comprising the steps of
i) Diluting the iodine solution in water,
ii) Sampling the diluted iodine solution into containers suitable for serial dilution,
iii) Homogenizing a suspension of *Eimeria oocysts*,
iv) Transferring the *Eimeria* suspension to the container with the iodine solution at a 1:1 ratio and continue with serial dilution using the iodine-based solution until the desired dilution,
v) Adding the diluted solution from iv) to a counting chamber,
vi) Quantifying the number of oocysts of *Eimeria* spp. in optical microscope.

3. Method, according to claim 1, **characterized in that** the proportion between the sample and the iodine-based solution in step a) is between 1:0.2 and 1:20.

4. Method, according to claim 2, **characterized in that** the proportion between the iodine-based solution and water in step i) is between 1:0.1 and 1:10, and the temperature is between 2 - 8 °C at the time of use.

5. Method according to any one of the preceding claims, **characterized in that** the iodine -based solution has a formulation of 0.4% to 3% of resublimated iodine; 1.8% to 5% potassium iodide; 70% to 85% alcohol; 25% to 35% purified water.

6. Method, according to any one of the preceding claims, **characterized in that** the starch particles and/or non-viable oocysts are stained by the iodine-based solution.

7. Method according to any one of the preceding claims, **characterized by** counting oocysts of *Eimeria* spp. stained and unstained and/or quantification of the number of *Eimeria* spp. be performed manually and/or through an image analysis system.

8. Method, according to any one of the preceding claims, **characterized in that** the counting chamber is a Neubouer, Fuchs Rosenthal, Petroff-Hausser, Sedgwick-Rafter or Burker chamber.

9. Method, according to any of the preceding claims, **characterized in that** it is used to verify the status of oocyst bulks, define the stability time of Active Pharmaceutical Inputs and vaccines, quality control of the manufacturing process of vaccines against coccidiosis, evaluation of improvements of production process, estimation of the level of vaccine response of the vaccine against coccidiosis *in vitro.*

## Amended claims

### Amended claims under Art. 19.1 PCT

1. *In vitro* method for evaluating the viability of *Eimeria oocysts* spp. comprising the steps of
a) Sampling a volume of suspension of oocysts of *Eimeria* spp. and mix with a volume of iodine-based solution,
b) Adding the mixture obtained in a) to a counting chamber,
c) Counting the oocysts of *Eimeria* spp. stained and unstained using an optical microscope, in order to determine the viability of the oocysts.

2. *In vitro* method for quantification of *Eimeria oocysts* spp. comprising the steps of
i) Diluting the iodine solution in water,
ii) Sampling the diluted iodine solution into containers suitable for serial dilution,
iii) Homogenizing a suspension of *Eimeria oocysts*,
iv) Transferring the *Eimeria* suspension to the container with the iodine solution at a 1:1 ratio and continue with serial dilution using the iodine-based solution until the desired dilution,
v) Adding the diluted solution from iv) to a counting chamber,
vi) Quantifying the number of oocysts of *Eimeria* spp. in optical microscope.

3. Method, according to claim 1, **characterized in that** the proportion between the sample and the iodine-based solution in step a) is between 1:0.2 and 1:20.

4. Method, according to claim 2, **characterized in that** the proportion between the iodine-based solution and water in step i) is between 1:0.1 and 1:10, and the temperature is between 2 - 8 °C at the time of use.

5. Method, according to any one of the preceding claims, **characterized in that** the iodine-based solution has a formulation of 0.4% to 3% of resublimated iodine; 1.8% to 5% potassium iodide; 70% to 85% alcohol; 25% to 35% purified water.

6. Method, according to any one of the preceding claims, **characterized in that** the differentiation of the starch particles and/or non-viable oocysts relative to viable oocysts is through dark staining acquired by the starch particles and/or non-viable oocysts when treated with the iodine-based solution as described in claim 5.

7. Method, according to any one of the preceding claims, **characterized by** counting oocysts of *Eimeria* spp. stained and unstained and/or quantification of the number of *Eimeria* spp. be performed manually and/or through an image analysis system.

8. Method, according to any one of the preceding claims, **characterized in that** the counting chamber is optionally any of Neubauer chamber, Fuchs Rosenthal chamber, Petroff-Hausser chamber, Sedgwick-Rafter chamber, or Burker chamber.

9. Method, according to any of the preceding claims, **characterized in that** it is used to verify the status of oocyst bulks, define the stability time of Active Pharmaceutical Inputs and vaccines, quality control of the manufacturing process of vaccines against coccidiosis, evaluation of improvements of production process, estimation of the level of vaccine response of the vaccine against coccidiosis *in vitro.*
